# EUROPÄISCHE PATENTSCHRIFT

(11) **EP 1 039 877 B1**
(45) Veröffentlichungstag und Bekanntmachung des Hinweises auf die Patenterteilung: **02.05.2003**
(21) Anmeldenummer: 98965764.8
(22) Anmeldetag: 04.12.1998
(51) Int. Cl.: A61K 7/13

(54) **VERFAHREN ZUR HERSTELLUNG VON HAARFÄRBEPRÄPARATEN MIT ERHÖHTER VISKOSITÄT**
METHOD FOR PRODUCING HAIR COLORING PREPARATIONS WITH IMPROVED VISCOSITY
PROCEDE DE PRODUCTION DE PREPARATIONS COLORANTES CAPILLAIRES A VISCOSITE ELEVEE

(30) Priorität: 13.12.1997 DE 19755491
(43) Veröffentlichungstag der Anmeldung: 04.10.2000
(73) Patentinhaber: Cognis Deutschland GmbH & Co. KG, 40589 Düsseldorf (DE)
(72) Erfinder: PITFIELD, Adrian, D-64753 Brombachtal-Kirchbrombach (DE); LORENZ, Heribert, D-64401 Grosz Bieberau (DE); GOLINSKI, Frank, D-64297 Darmstadt (DE); KAHRE, Jörg, D-42799 Leichlingen (DE); FÖRSTER, Thomas, D-40699 Erkrath (DE); SUMSER, Markus, D-44625 Herne (DE); WADLE, Armin, D-40699 Erkrath (DE)
(86) Internationale Anmeldenummer: EP9807903
(87) Internationale Veröffentlichungsnummer: WO99030676

(56) Entgegenhaltungen:
- WO-A-93/23006
- WO-A-98/51267
- CH-A- 188 988
- DE-A- 2 736 067
- DE-A- 4 332 965

## Beschreibung

### Gebiet der Erfindung

Die Erfindung betrifft ein Verfahren zur Kaltherstellung von Haarfärbepräparaten, vorzugsweise Färbe-cremes, bei dem man tensidische Fettalkoholdispersionen einsetzt.

### Stand der Technik

Zur Herstellung von Haarfärbepräparaten, wie z.B. Cremes vom Öl-in-Wasser-Typ, werden zur Einstellung der Viskosität üblicherweise Fettalkohole eingesetzt. Die Herstellung der Emulsionen erfolgt dabei nach dem Heißverfahren, d.h. die Bestandteile werden oberhalb des Schmelzpunktes der am höchsten schmelzenden Komponente vermischt und dann unter intensivem Homogenisieren langsam abgekühlt. Aus Gründen der Wirtschaftlichkeit ist es indes wünschenswert, solche Zubereitungen auf kaltem Wege, beispielsweise unter Einsatz der bekannten PIT- oder Mikroemulsions-Technologie herzustellen. Hierbei tritt jedoch das Problem auf, daß die Einstellung von hohen Viskositäten nur sehr schwer gelingt, insbesondere dann, wenn es darum geht unter Einsatz von Fettalkoholen lamellare Gele herzustellen. Die internationale Patentanmeldung **WO 93/23006 A1** sowie die deutsche Patentanmeldung **DE 4332965 A1** beschreiben cremeförmige oxidations Haarfärbemittel aus einer Emulsion die ein Fettalkohol ein Emulgator einer Ölkomponente sowie Wasser enthält.

Die Aufgabe der Erfindung hat folglich darin bestanden, ein Verfahren zur Kaltherstellung von Haarfärbepräparaten, insbesondere Cremes vom O/W-Typ, zur Verfügung zu stellen, mit dessen Hilfe sich auch lamellare Gele mit besonderer Lagerstabilität herstellen lassen.

### Beschreibung der Erfindung

Gegenstand der Erfindung ist ein Verfahren zur Herstellung von Haarfärbepräparaten mit erhöhter Vis-kosität, bei dem man wäßrige tensidische Fettalkoholdispersionen mit Ölkomponenten und Haarfarbstoffen kalt verrührt.

Überraschenderweise wurde gefunden, daß sich O/W-Cremezubereitungen für das Färben von Haaren auch auf kaltem Wege herstellen lassen, wenn man zur Einstellung der Konsistenz tensidische Fettalkoholdispersionen, vorzugsweise Fettalkoholmikrodispersionen einsetzt. Die resultierenden Mittel liegen dabei in Form lamellarer Gele vor, die sich durch eine besonders hohe Stabilität auch bei Temperaturlagerung auszeichnen.

### Fettalkohole

Die im Sinne des erfindungsgemäßen Verfahrens einzusetzenden Dispersionen können Fettalkohole der Formel **(I)** enthatten,

**R**^{**1**}**OH (I)**

in der R¹ für einen linearen oder verzweigten, gesättigten oder ungesättigten Alkylrest mit 6 bis 30 Kohlenstoffatomen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkem-, Cetearyl- oder Talgfettalkohol. Der Anteil der Fettalkohole an den Dispersionen kann dabei 5 bis 50 und vorzugsweise 25 bis 40 Gew.-% betragen.

### Tenside

Die im Sinne des erfindungsgemäßen Verfahrens einzusetzenden Fettalkoholdispersionen können anionische, nichtionische, kationische und/oder amphotere bzw. zwitterionische Tenside enthalten. Typische Beispiele für **anionische Tenside** sind Seifen, Alkylbenzofsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureestersulfate, insbesondere Laurinsäure+1EO-sulfat, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologen Verteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Der Anteil der Tenside an den Fettalkoholdispersionen kann 0,1 bis 10 und vorzugsweise 0,5 bis 5 Gew.-% betragen.

### Fettalkoholdispersionen

Die Herstellung der Fettalkoholdispersionen kann beispielsweise nach dem Mikrodispersionsverfahren erfolgen. Mikrodispersionen stellen optisch isotrope, thermodynamisch stabile Systeme dar, die eine wasserunlösliche Ölkomponente (hier: Fettalkohol), Dispergatoren - vorzugsweise Alkylglucoside - und Wasser enthalten. Das klare bzw. transparente Aussehen der Mikrodispersionen ist eine Folge der geringen Teilchengröße der dispergierten Emulsionströpfchen. In diesem Zusammenhang hat sich erwiesen, daß Fettalkoholmikrodispersionen einen besonders vorteilhaften Einfluß auf die Herstellung und Lagerstabilität der resultierenden Färbepräparate besitzen. Vorzugsweise werden Dispersionen eingesetzt, die eine Teilchengröße kleiner 50 µm, insbesondere kleiner 20 µm und besonders bevorzugt kleiner 10 µm aufweisen. Übersichten zu Herstellung und Anwendung von Mikrodispersionen finden sich von H.Eicke in **SÖFW-Journal, 118, 311 (1992)** und Th.Förster et al. in SÖFW-Journal, **122, 746 (1996);** des weiteren sei auf die Druckschriften **DE-A1 4411557** (Henkel) und **EP-A1 0687206** (L'Oréal) verwiesen.

### Ölkomponenten

Als **Ölkomponenten** kommen beispielsweise Guerbetalkohole auf Basis von Fettalkoholen, mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestem mit Polyolen, Siliconöle und/oder aliphatische bzw. naphthenische Kohlenwasserstoffe in Betracht.

### Haarfarbstoffe

Als Haarfarbstoffe kommen dabei beispielsweise **direktziehende Farbstoffe,** z.B. aus der Gruppe der Nitrophenylendiamine, Nitroaminophenole, Anthrachinone oder. Indophenole in Betracht, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Basic Brown 17, Pikraminsäure und Rodol 9 R bekannten Verbindungen sowie 4-Amino-2-nitrodiphenylamin-2'carbonsäure, 6-Nitro-1,2,3,4-tetrahydrochinoxalin, (N-2,3-Dihydroxypropyl-2-nitro-4-trifluormethyl)-aminobenzol und 4-N-Ethyl-1,4-bis(2'-hydroxyethylamino)-2-nitrobenzol-hydrochlorid. Weiterhin können den Emulsionen auch in der Natur vorkommende Farbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel zugesetzt werden.

Neben den Direktziehern können den Emulsionen auch **Oxidationsfarbstoffe,** bestehend aus Entwickler- und Kupplerkomponente zugesetzt werden. Als Entwicklerkomponenten werden beispielsweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind u.a. p-Toluylendiamin, p-Aminophenol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5 und 4-Amino-3-methyl-phenol, 2-(2-Hydroxyethyl)-1,4-aminobenzol und 2,4,5,6-Tetraaminopyrimidin. Als Kupplerkomponenten werden in der Regel m-Phenylendiaminderivate, Naphthole, Resorcin und Resorcinderivate, Pyrazo-lone, m-Aminophenole sowie Pyridin-Derivate verwendet. Als Kupplersubstanzen eignen sich insbe-sondere 1-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 2-Chlor-6-methyl-3-aminophenol, 2-Methylresorcin, 2,5-Dimethylresorcin, 2,6-Dihydroxypyridin und 2,6-Diaminopyridin.

Bezüglich weiterer Farbstoffkomponenten wird ausdrücklich auf die Colipa-Liste, herausgegeben vom Industrieverband Körperpflege und Waschmittel, Frankfurt, Bezug genommen. Eine Übersicht zu geeigneten Farbstoffen ist weiterhin der Publikation **"Kosmetische Färbemittel" der Farbstoffkommission der Deutschen Forschungsgemeinschaft, Verlag Chemie, Weinheim, 1984, S.81-106** zu entnehmen. Die Haarfarben können in Mengen von 0,1 bis 15, vorzugsweise 0,5 bis 10 und insbesondere 1 bis 5 Gew.-% - bezogen auf die Haarfärbepräparate - enthalten sein.

### Polymere Verdickungsmittel

In einer weiteren bevorzugten Ausführungsform der Erfindung enthalten die Haarfärbepräparate neben den tensidischen Fettalkoholdispersionen polymere Verdickungsmittel, wie z.B. Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycol-mono- und -diester von Fettsäuren, Polyacrylate, (z.B. Carbopole® von Goodrich oder Synthalene® von Sigma), Polyacrylamide, Polyvinylalkohol und Polyvinylpyrrolidon. Die Polymeren können in Mengen von 0,1 bis 5, vorzugsweise 0,5 bis 2 Gew.-% - bezogen auf die Haarfärbepräparate - eingesetzt werden.

### Gewerbliche Anwendbarkeit

Unter Einsatz der tensidischen Fettalkoholdispersionen gelingt es auch auf kaltem Wege, d.h. bei Temperaturen im Bereich von 18 bis 25°C Haarfärbepräparate herzustellen, die in Form lamellarer Gele vorliegen. Daher können tensidische Fettalkoholdispersionen, vorzugsweise Fettalkoholmikrodispersionen, als Konsistenzgeber zur Herstellung von Haarfärbepräparaten, vorzugsweise von O/W-Färbecremes, eingesetzt werden, in denen sie in Mengen von 10 bis 70 und vorzugsweise 25 bis 35 Gew.-% enthalten sein können.

### Weitere Hilfs- und Zusatzstoffe

Bei den erfindungsgemäßen Haarfärbepräparaten handelt es sich vorzugsweise um O/W-Cremes, die als weitere Hilfs- und Zusatzstoffe, Co-Emulgatoren, Überfettungsmittel, Perlglanzwachse, Stabilisatoren, weitere Konsistenzgeber, Elektrolytsalze, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Parfümöle und dergleichen enthalten können.

Die in den Fettalkoholdispersionen enthaltenen Tenside, die dort als Dispergatoren wirken, können in den Endzubereitungen gleichfalls als Emulgatoren dienen, Daneben können den Haarfärbepräparaten auch weitere Tenside bzw. **Co-Emulgatoren** zugesetzt werden, wie z.B.:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als weitere **Konsistenzgeber** kommen neben den Fettalkoholen vor allem Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinyl-pyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P.Finkel in **SÖFW-Journal 122, 543 (1996)** zu entnehmen.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.

### Beispiele

**Beispiele 1 bis 4, Vergleichsbeispiel V1.** Zur Herstellung der erfindungsgemäßen Cremes 1 bis 4 wurde jeweils eine etwa 30 Gew.-%ige Dispersion von Cetylstearylalkohol in Wasser eingesetzt, die als Stabilisatoren jeweils 1 Gew.-% - bezogen auf die Dispersion - Sodium Lauryl Sulfate (D1), Sodium Laureth Sulfate (D2) bzw. Coco Glucosides (D3,D4) enthielt. Die Herstellung der Dispersionen erfolgte nach dem Mikrodispersions-Verfahren. Die Dispersion D1 wies eine mittlere Teilchengröße von 55 µm auf, die Dispersionen D2 und D3 von 40 µm und die Dispersion D4 von 10 µm. Im Fall der Vergleichscreme V1 wurde der Cetylstearylalkohol direkt, also nicht als wäßrige tensidische Dispersion eingerührt. Die Herstellung der fünf O/W-Cremes erfolgte durch Homogenisieren der Komponenten bei 20°C. Die Viskosität wurde unmittelbar sowie nach Lagerung über einen Zeitraum von 4 Wochen bei 40°C nach der Brookfield-Methode bestimmt (10 Upm, Spindel 1). Die Ergebnisse sind in Tabelle 1 zusammengefaßt:

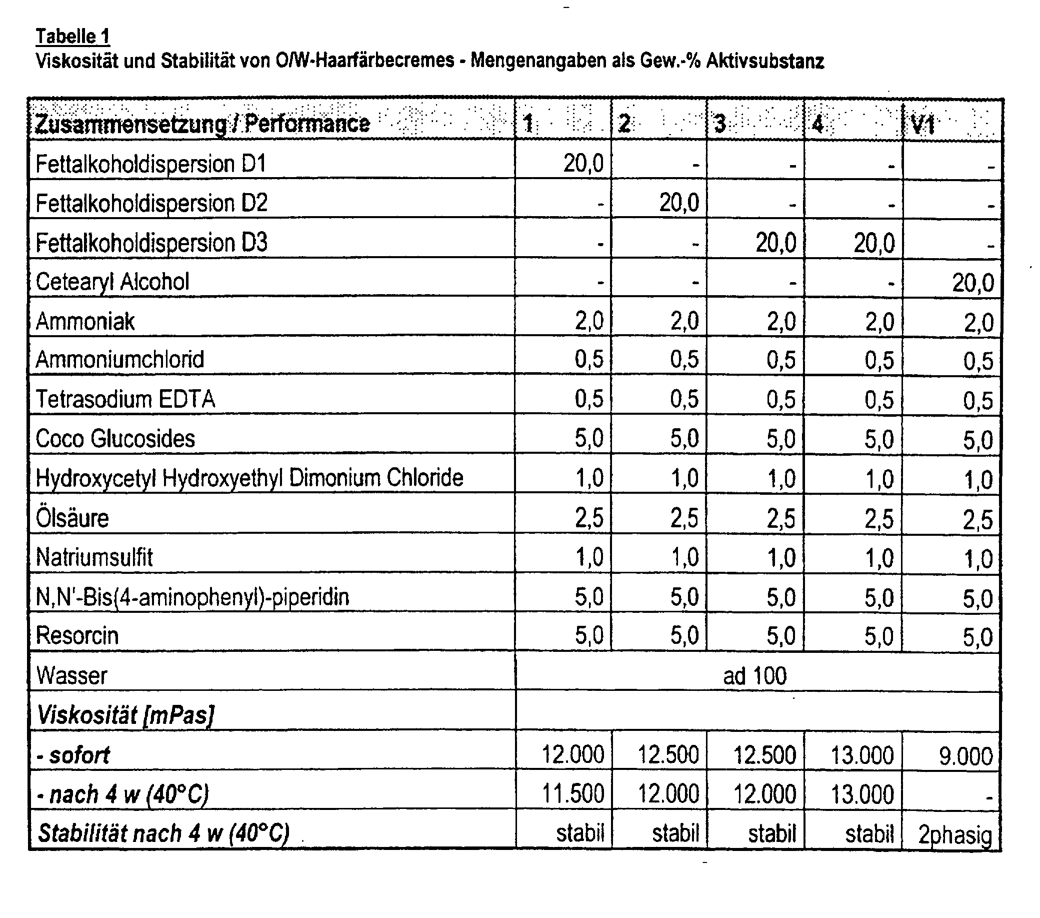

## Patentansprüche

1. Verfahren zur Herstellung von Haarfärbepräparaten mit erhöhter Viskosität, bei dem man wäßrige tensidische Fettalkoholdispersionen mit Ölkomponenten und Haarfarbstoffen kalt verrührt.

2. Verfahren nach Anspruch 1, **dadurch gekennzeichnet, daß** man Dispersionen einsetzt, die Fettalkohole der Formel **(I)** enthalten,
**R**^{**1**}**OH (I)**
in der R¹ für einen linearen oder verzweigten, gesättigten oder ungesättigten Alkylrest mit 6 bis 30 Kohlenstoffatomen steht.

3. Verfahren nach den Ansprüchen 1 und 2, **dadurch gekennzeichnet, daß** man Dispersionen einsetzt, die die Fettalkohole in Mengen von 5 bis 50 Gew.-% - bezogen auf die Dispersionen - enthalten.

4. Verfahren nach den Ansprüchen 1 bis 3, **dadurch gekennzeichnet, daß** man Dispersionen einsetzt, die anionische, nichtionische, kationische und/oder amphotere bzw. zwitterionische Tenside enthalten.

5. Verfahren nach den Ansprüchen 1 bis 4, **dadurch gekennzeichnet, daß** man Dispersionen einsetzt, die die Tenside in Mengen von 0,1 bis 10 Gew.-% - bezogen auf die Dispersionen enthalten.

6. Verfahren nach den Ansprüchen 1 bis 5, **dadurch gekennzeichnet, daß** man Dispersionen einsetzt, die eine Teilchengröße kleiner 50 µm aufweisen.

7. Verfahren nach den Ansprüchen 1 bis 3, **dadurch gekennzeichnet, daß** man Ölkomponenten einsetzt, die ausgewählt sind aus der Gruppe, die gebildet wird von Guerbetalkoholen auf Basis von Fettalkoholen mit 6 bis 18 Kohlenstoffatomen, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen undloder Guerbetalkoholen mit aromatischen Carbonsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Ölen, verzweigten primären Alkoholen, substituierten Cyclohexanen, linearen C₆-C₂₂-Fettalkoholcarbonaten, Guerbetcarbonaten, Estem der Benzoesäure mit linearen und/ oder verzweigten C₆-C₂₂-Alkoholen, Dialkylethem, Ringöffnungsprodukten von epoxidierten Pett-säureestem mit Polyolen, Siliconölen und/oder aliphatischen bzw. naphthenischen Kohlenwas-serstoffen.

8. Verfahren nach den Ansprüchen 1 bis 7, **dadurch gekennzeichnet, daß** man direktziehende Farbstoffe und/oder Oxidationsfarbstoffe einsetzt.

9. Verfahren nach den Ansprüchen 1 bis 8, **dadurch gekennzeichnet, daß** man polymere Verdickungsmittel einsetzt.

## Claims

1. A method for producing hair coloring preparations with improved viscosity, in which aqueous surface-active fatty alcohol dispersions are stirred up with oil components and hair dyes in the cold.

2. The method as claimed in claim 1, wherein dispersions are used which comprise fatty alcohols of the formula **(I)**
**R**^{**1**}**OH (I)**
in which R¹ is a linear or branched, saturated or unsaturated alkyl radical having 6 to 30 carbon atoms.

3. The method as claimed in claims 1 and 2, wherein dispersions are used which comprise the fatty alcohols in amounts of from 5 to 50% by weight, based on the dispersions.

4. The method as claimed in claims 1 to 3, wherein dispersions are used which comprise anionic, nonionic, cationic and/or amphoteric or zwitterionic surfactants.

5. The method as claimed in claims 1 to 4, wherein dispersions are used which comprise the surfactants in amounts of from 0.1 to 10% by weight, based on the dispersions.

6. The method as claimed in claims 1 to 5, wherein dispersions are used which have a particle size of less than 50 µm.

7. The method as claimed in claims 1 to 3, wherein oil components are used which are chosen from the group formed from Guerbet alcohols based on fatty alcohols having 6 to 18 carbon atoms, esters of linear C₆-C₂₂-fatty acids with linear C₆-C₂₂-fatty alcohols, esters of branched C₆-C₁₃-carboxylic acids with linear C₆-C₂₂-fatty alcohols, esters of linear C₆-C₂₂-fatty acids with branched alcohols, esters of linear and/or branched fatty acids with polyhydric alcohols and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear C₆-C₂₂-fatty alcohol carbonates, Guerbet carbonates, esters of benzoic acid with linear and/or branched C₆-C₂₂-alcohols, dialkyl ethers, ring-opening products of epoxidized fatty acid esters with polyols, silicone oils and/or aliphatic or naphthenic hydrocarbons.

8. The method as claimed in claims 1 to 7, wherein direct dyes and/or oxidation dyes are used.

9. The method as claimed in claims 1 to 8, wherein polymeric thickeners are used.

## Revendications

1. Procédé de production de préparations de teinture pour cheveux ayant une viscosité accrue, dans lequel on mélange à froid des dispersions aqueuses d'alcool gras contenant des agents tensioactifs avec des composants huileux et des colorants pour les cheveux.

2. Procédé selon la revendication 1,
**caractérisé en ce qu'**
on met en oeuvre des dispersions qui renferment des alcools gras de formule (I) :
R¹OH (I)
dans laquelle R¹ représente un reste alkyle linéaire ou ramifié, saturé ou non saturé, ayant de 6 à 30 atomes de carbone.

3. procédé selon les revendications 1 et 2,
**caractérisé en ce qu'**
on met en oeuvre des dispersions qui renferment les alcools gras en quantités de 5 à 50 % en poids, rapporté aux dispersions.

4. Procédé selon les revendications 1 à 3,
**caractérisé en ce qu'**
on met en oeuvre des dispersions qui renferment des agents tensioactifs anioniques, non ioniques, cationiques et/ou amphotères ou zwitterioniques.

5. Procédé selon les revendications 1 à 4,
**caractérisé en ce qu'**
on met en oeuvre des dispersions qui renferment les agents tensioactifs en quantités allant de 0,1 à 10 % en poids, rapporté aux dispersions.

6. procédé selon les revendications 1 à 5,
**caractérisé en ce qu'**
on met en oeuvre des dispersions qui possèdent une taille de particules inférieure à 50 µm.

7. Procédé selon les revendications 1 à 3,
**caractérisé en ce qu'**
on met en oeuvre des composants huileux choisis dans le groupe formé des alcools de Guerbet à base d'alcools gras ayant de 6 à 18 atomes de carbone, des esters d'acides gras linéaires en C₆-C₂₂ avec des alcools gras linéaires en C₆-C₂₂, des esters d'acides carboxyliques en C₆-C₁₃ ramifiés avec des alcools gras en C₆-C₂₂ linéaires, des esters d'acides gras en C₆-C₂₂ linéaires avec des alcools ramifiés, des esters d'acides gras linéaires et/ou ramifiés avec des alcools plurifonctionnels et/ou des alcools de Guerbet, des triglycérides à base d'acides gras en C₆-C₁₀, des mélanges liquides de mono-, di-, et triglycérides à base d'acides gras en C₆-C₁₈, des esters d'alcools gras en C₆-C₂₂ et/ou d'alcools de Guerbet avec des acides carboxyliques aromatiques, des esters d'acides dicarboxyliques en C₂-C₁₂ avec des alcools linéaires ou ramifiés ayant de 1 à 22 atomes de carbone ou des polyols ayant de 2 à 10 atomes de carbone et de 2 à 6 groupes hydroxyle, des huiles végétales, des alcools primaires ramifiés, des cyclohexanes substitués, des carbonates d'alcools gras en C₆-C₂₂ linéaires, des carbonates de Guerbet, des esters d'acide benzoïque avec des alcools linéaires et/ou ramifiés en C₆-C₂₂, des éthers de dialkyle, des produits d'ouverture de cycle d'esters d'acides gras époxydés, par des polyols, des huiles de silicone et/ou des hydrocarbures aliphatiques ou naphténiques.

8. Procédé selon les revendications 1 à 7,
**caractérisé en ce qu'**
on met en oeuvre des colorants à action directe et/ou des produits colorants par oxydation.

9. Procédé selon les revendications 1 à 8,
**caractérisé en ce qu'**
on met en oeuvre des agents épaississants polymères.
